# EUROPEAN PATENT APPLICATION

(11) **EP 3 603 649 A1**
(43) Date of publication of application: **05.02.2020**
(21) Application number: 18306038.3
(22) Date of filing: 31.07.2018
(51) Int. Cl.: A61K 31/724, A61K 9/00, A61P 25/28

(54) **METHOD FOR TREATING PRION DISEASES**

(71) Applicant: Medday Pharmaceuticals, 75008 Paris (FR); Institut du Cerveau et de la Moelle Epiniere, 75013 Paris (FR)
(72) Inventor: The designation of the inventor has not yet been filed
(74) Representative: Flesselles, Bruno F.G.

(57) **Abstract**

The invention relates to the treatment of a prion neurodegenerative disease by nasal administration of 2,6-di-O-methyl-beta-cyclodextrin (DIMEB).

## Description

The invention relates to the treatment of a prion neurodegenerative disease in particular by nasal administration of cyclodextrin.

Prion diseases, also called transmissible spongiform encephalopathies (TSEs) are a family of rare progressive neurodegenerative disorders that affect both humans and animals. They are characterized by a long clinically silent incubation phase, characteristic spongiform changes associated with neuronal loss, and a failure to induce inflammatory response.

The causative agents of TSEs are believed to be prions, which are proteinaceous infectious particles and refer to abnormal, pathogenic agents that are transmissible. The prion agent consists of PrP^{Sc} (for Scrapie prion protein), which is an abnormal isoform of the host-encoded prion cellular protein, PrP^{C} (for the Cellular form of the protein). Prion propagation is considered to occur in a two-step process in which aggregates of PrP^{Sc} bind new PrP^{C} monomers and then induce their conformational change into PrP^{Sc}. The occurrence of different TSE strains has been described in several species. These strains correspond to different PrP^{Sc} conformations, according to the prion hypothesis. Prion strain transmission among different species seems to be driven by the differences in the PrP amino acid sequences and by the prion strain being transmitted. Prion diseases are usually rapidly progressive and always fatal.

Human forms of prion disease include Creutzfeldt-Jakob disease (CJD), fatal familial insomnia (FFI), Gerstmann-Straussler-Scheinker syndrome (GSS), kuru and variably protease-sensitive prionopathy (VPSPr). It is to be noted that the diseases can be sporadic (Creutzfeldt-Jakob disease), genetic (genetic Creutzfeldt-Jakok disease, Gerstmann-Straussler-Scheinker syndrome, and fatal familial insomnia), and acquired (kuru, variant Creutzfeldt-Jakob disease, and iatrogenic Creutzfeldt-Jakob disease).

There is currently no disease-modifying treatment for these kinds of diseases.

Cyclodextrins (CD) are cyclic oligosaccharides forming "ring" structures with a hydrophobic inner and hydrophilic outer surface. These compounds are used as formulation vehicles to increase the quantities of drugs, such as hormones and vitamins, and for solubilization in aqueous media. A significant number of cyclodextrins are approved for use in humans and are used in a variety of drugs or in the food industry. Cyclodextrins, with their lipophilic internal cavities and hydrophilic external surfaces, are likely to interact with many molecules to form non-covalent inclusion complexes. They are cyclic oligosaccharides that contain at least 6 D-(+) glucopyranose units attached by glucosidic bonds. The three natural cyclodextrins, alpha-, beta-, and gamma-CDs (with 6, 7, or 8 glucose units respectively), differ in ring size and solubility.

Dimethyl-beta-cyclodextrin (DIMEB), also called heptakis(2,6-O-dimethyl)beta-cyclodextrin, has been used in pharmaceutical compositions, in particular as a stabilizer of molecules (Saleh et al, J Pharm Belg. 1993 Sep-Oct;48(5):383-8) or as a parenteral drug carrier (Szejtli, Journal of Inclusion Phenomena (1983) 1: 135).

It or other cyclodextrins have also been proposed in nasal formulations such as nasal insulin (Ahsan et al, Pharm Res. 2001 May;18(5):608-14) or peptides (Agu et al, Int J Pharm. 2002 Apr 26;237(1-2):179-91) formulations. Table 3 of Tiwari et al (J Pharm Bioallied Sci. 2010 Apr-Jun; 2(2): 72-79) summarizes some of the uses of cyclodextrins in formulations intended for nasal administrations.

WO 2016/16168772 describes and claims the use of cyclodextrins, in particular by the nasal route and for the treatment of various diseases. The examples only describe the use of 2 hydroxypropyl beta-cyclodextrin *in vitro* in a physiological process (removal of lipofuscin from cells) that is not known as being linked to a prion disease.

The use of cyclodextrins for the treatment of patients with neurodegenerative diseases, and in particular prion diseases is limited by the fact that these compounds do generally not cross the blood-brain barrier.

For example, *in vivo* studies in mice showed that CDs did not cross the blood-brain barrier (Calias P, Curr Pharm Des. 2017 Oct 19). These studies suggested a potential for these oligosaccharides to moderately impact CNS manifestations when administered subcutaneously or intraperitoneally at very high doses. However, safety concerns regarding pulmonary toxicity were raised.

In mouse models of type C Niemann Pick disease, an extremely high dose of cyclodextrin (3 different 2-hydroxypropyl-beta-cyclodextrins) is administered: a 3 times/week dose of 500 mg/kg cyclodextrin is administered by intraperitoneal injection (Camargo et al. 2001, Life Sci 70(2):131-42). Treatment with two HPBCDs, with varying levels of 2-hydroxypropyl substitution, had effects in delaying neurological symptoms and in decreasing liver cholesterol storage while a third HPBCD was without effect. Conversely, a single subcutaneous dose of 4000 mg/kg of a 20% cyclodextrin solution in 7-day-old NPC mice restored lysosomal cholesterol transport, significantly improved liver dysfunction, decreased neuronal degeneration, and prolonged survival (Liu et al., 2009, Proc Natl Acad Sci USA 106:2377-2382; Davidson et al., 2009, PLoS One 4(9):e6951; Aqul et al., 2011, J Neurosci.;31(25):9404-13). Subcutaneous administration of a 4000 mg/kg subcutaneous dose of 20% cyclodextrin solution every two days was the best treatment. However, cyclodextrin administered intracerebroventricularly at a dose of 23 mg/kg/day (more than 150-fold lower) was more effective (Aqul et al., 2011, *op. cit.*)*.* These doses are extremely high. Furthermore, Shityakov et al have shown that toxicity of cyclodextrins on endothelial cells of the BBB depends on the type of modification to the molecule trimethyl-β-cyclodextrin (TRIMEB) being more toxic than α-cyclodextrin (a-CD), and hydroxypropyl-β-cyclodextrin (HPβCD) being the less toxic and the only proposed molecule for therapeutic uses.

Theoretically, cyclodextrins could be administered directly into the central nervous system by intra-ventricular or intraparenchymal injections; however, for treatments requiring multiple injections, these methods are not appropriate because they are invasive, risky, expensive and require surgical expertise.

It is thus necessary to develop a method of delivering cyclodextrin to the brain, to enable it to pass through the blood-brain barrier and repeat administration in long-term treatment.

The inventors proposed to use dimethyl-β-cyclodextrin to treat neurodegenerative prion diseases, in particular by nasal administration. Such way of administration would allow the cyclodextrin to cross the BBB and access the brain. It is thus proposed to have the cyclodextrin administered directly into the central nervous system and to avoid the systemic circulation, while simplifying the administration mode to increase ease and safety.

In the present application, cyclodextrin is not used as an adjuvant for a therapeutically active molecule, but as the therapeutic molecule. It is also to be noted that the use envisaged is a long-term use for the treatment of a chronic disease and that it is important to ensure that there is no inflammation due to prolonged administration of cyclodextrin when it is used intranasally. Consequently, there may be some risks of inflammation, depending on the preservative chosen in the formulation. The formulations herein disclosed most preferably don't contain any other active ingredient or medicament, intended for the treatment of the diseases herein contemplated. The compositions thus don't use cyclodextrin as a vehicle for drug delivery through the BBB.

It is thus proposed to use an intranasal delivery system, as an alternative to invasive systems, which allows to pass through the Blood-Brain Barrier, and to send the molecule into the central nervous system *via* the olfactory and trigeminal nerves that are present in the nasal openings. The anatomical regions involving vascularization, cerebrospinal fluid, and the lymphatic system allow the transport of molecules from the nasal cavity to the central nervous system (Dhuria et al., 2010, J Pharm Sci.;99(4):1654-73). The neural projections of olfactory neurons extend across multiple regions of the brain, including the olfactory tract, olfactory anterior nucleus, piriform cortex, amygdala and hypothalamus. These regions are of particular interest for the treatment of prion diseases.

The invention thus relates to 2,6-di-O-methyl-beta-cyclodextrin (DIMEB) for its use for the treatment of a neurodegenerative disease, in particular by nasal administration, wherein the neurodegenerative disease is a prion disease.

Indeed, the examples clearly show that such product can be used to inhibit aggregation and accumulation of the prion protein.

The prion disease is preferably selected from the group consisting of Creutzfeldt-Jakob disease (CJD), iatrogenic Creutzfeldt-Jakob disease (iCJD), variant Creutzfeldt-Jakob disease (vCJD), inherited or familial Creutzfeldt-Jakob disease (fCJD), sporadic Creutzfeldt-Jakob disease (sCJD), Gerstmann-Straussler-Scheinker syndrome (GSS), familial fatal insomnia (FFI), Kuru, familial spongiform encephalopathy.

### Composition of the medicament

In a specific embodiment, the nasal composition contains only (2,6-di-O-methyl)-beta-cyclodextrin and water without any other component.

In another embodiment, the nasal composition contains (2,6-di-O-methyl)-beta-cyclodextrin dissolved in an isotonic solution. An isotonic solution corresponds to 0.9% by weight (or 9 g/L) of sodium chloride in aqueous solution.

The solution containing the cyclodextrin is with or without preservatives or excipients.

Preferably, the pH of the solution is comprised between 5 and 8. It is preferred when it is around pH 6 (i.e. comprised between 5.6 and 6.4, more preferably between 5.8 and 6.2, or 5.9 and 6.1).

The osmotic concentration is preferably comprised between 300 and 350 mOsm/L, and more preferably 330 mOsm/L.

In another embodiment, the nasal composition further contains preservatives. In particular, the preservative may be EDTA. It is to be noted that one should choose a preservative that is not inhibited by cyclodextrin. It is further to be noted that the preservatives or excipients added to the composition must be suitable for nasal administration. Since these products may have local toxicity, care should be taken to minimize degradation of the nasal mucosa. This can be checked on animals (e.g. mice, rats or rabbits).

The cyclodextrin may be used in a potassium sodium phosphate buffer 10 mM. Such buffer, with pH and osmolarity adjusted as mentioned above, is suitable to achieve the goal of allowing cyclodextrin to penetrate within the olfactory nervous system, while minimizing degradation of the nasal tissue environment. It is however to be noted that the patients envisaged therein may generally have a weakened sense of smell and that some nasal inflammation may thus be acceptable.

The composition shall not contain any other element that (2,6-di-O-methyl)-beta-cyclodextrin and a potential preservative (such as EDTA) in an isotonic buffer as disclosed above.

### Administration of the medicament

The DIMEB is to be administered nasally to the patient in such a way as to reach the olfactory mucosa, in order to cross the BBB. By conducting extensive experiments, the applicant has determined that this is most favorably obtained when the patient is lying on its back (i.e. is in supine position) during nasal administration. This position of the patient maximizes the amount of product reaching the olfactory mucosa and makes it possible to maximize the amount of product entering the brain.

It is also preferred when the angle of the dispensing device is about 60° relative to the vertical line (line perpendicular to the horizontal plane determined by the lying body (coronal plane). It is further preferred when the dispensing device is introduced in the nostril with an angle of about 5° relative to the vertical plane (median or midsagittal plane).

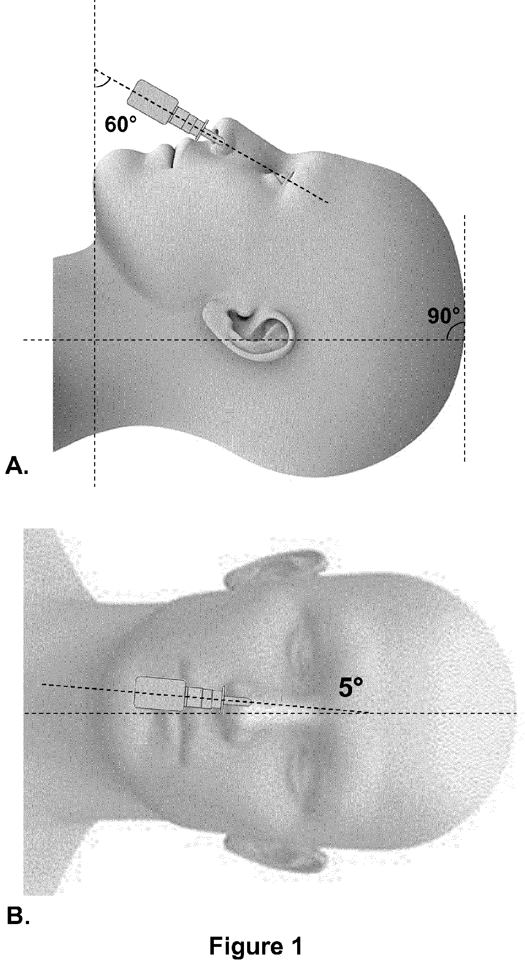

Figure 1 shows the optimal delivery mode of DIMEB in the patient's nostrils.

It is to be noted that specific devices for nasal administration have been developed, such as the optinose product ONZETRA® Xsail® (Avanir Pharmaceuticals, Inc), which is not a nasal spray or inhaler, but is a device comprising two tubular parts, one to be put in the mouth and the other in the nose, and the medication being delivered in the nose when the patient blows with his mouth. This device is said to be able to deliver medications to the posterior part of the nasal cavity. However, it is not suitable for patients with one of the diseases listed above, which are generally not able to blow in such a way to have delivery of the drug. Furthermore, there is a risk of lack of control of the amount of the drug delivered to the patient.

In contrast, the present device (nasal spray) and the preferred method of delivery (in supine position) make it possible to deliver the proper and controlled amount of (2,6-di-O-methyl)-beta-cyclodextrin, at the right location in the nasal cavity and to patients that have weakened physical abilities. It is preferable when another person helps the patient for administration of the composition, but the proposed administration, especially when the patient lies in the supine position doesn't necessitate any active action from the patient (such as breath or sniffing), as the droplets will reach the desired nasal region by themselves.

### Delivery of the (2,6-di-O-methyl)-beta-cyclodextrin

The (2,6-di-O-methyl)-beta-cyclodextrin is preferably delivered within the nasal cavity though spraying. Consequently, the (2,6-di-O-methyl)-beta-cyclodextrin is preferably contained in a spray bottle (where dispensing is powered by the user's efforts) or preferably in a spray can (where the user actuates a valve and product is dispensed). It is preferred to use a spray can in order to be able to control the amount of product dispensed at each take. Indeed, in the frame of a long-term treatment, it is preferred to make sure that the amount (volume) received every day or at every take remains constant.

Spray cans (or aerosol sprays) are known in the art. This is a type of dispensing system which creates an aerosol mist of liquid particles.

Among these sprays cans, one preferably uses cans of the bag-in-can (or BOV, bag-on-valve technology) system in which the product is separated from the pressurizing agent with a hermetically sealed, multi-layered laminated pouch, which maintains complete formulation integrity so only pure product is dispensed. This device consists of an aerosol valve with a welded bag. The product is placed inside the bag and the propellant is filled in the space between the flexible bag and the rigid can. When the spray button is pressed, the propellant simply squeezes the bag, and the product is squeezed out of the bag by the compressed air/nitrogen.

Among such products one can use the bag on valve system developed by Aptar pharma (Aptar group, Crystal Lake, IL, USA). Such dispensing devices are suitable for a 360° use, are continuous dispensing systems, with no back contamination and a propellant that is compressed air or nitrogen (separated from the product).

One can also cite products from Gaplast, of the same technology and disclosed in particular in US 9199785.

In brief, such technology makes use of a substantially rigid outer container and an easily deformable inner bag (containing the product to be dispensed).

In such technology, when the product is dispensed e.g. with the help of a pump, the volume of the inner bag is decreasing, and air enters from the surroundings of the container into the space between inner bag and outer container for the purpose of pressure compensation. The inner bag is compressed and becomes increasingly flatter.

One can also cite the CPS technology developed by Aptar, which delivers the drug whatever the position of the pump and of the container.

### Amount and concentration

It is intended to deliver a daily amount of (2,6-di-O-methyl)-beta-cyclodextrin comprised between 5 and 50 mg.

In one embodiment, the daily delivered amount is around 8 mg (around meaning ± 5 %). In another embodiment, the daily delivered amount is around 16 mg. In another embodiment, the daily delivered amount is around 24 mg.

The dosage delivered by each spray is preferably around 100 µl. The concentration of the (2,6-di-O-methyl)-beta-cyclodextrin in the container may depend on the number daily of takes by the patients.

For instance, if the patients take only two takes of the drug (one spray in each nostril) and the expected daily amount is 16 mg, the concentration would be 80 mg/ml. In the case the patients take two shots in each nostril every day, the concentration would be 40 mg/ml.

It is preferred when the patients take two shots in each nostril every day, and preferably when they take one shots in each nostril in the morning and one shot in each nostril in the evening. The treatment is expected to be given to the patient for continuous long periods of times, and generally more than one year. Since prion diseases are chronic diseases, the treatment should not be stopped once started. Furthermore, it is believed that the effect of the therapy (improvement in functional capacities of the patient or stop or decrease of neurodegeneration in the patient) will be observed after a long period of administration.

Knowing the expected dosage required by the patient and the amount delivered by each spray thus makes it possible to calculate the concentration of the product within the container.

The invention also relates to a device for nasally administering (2,6-di-O-methyl)-beta-cyclodextrin, comprising a container filled with a liquid composition containing (2,6-di-O-methyl)-beta-cyclodextrin, a nasal tip allowing delivery of the composition through spraying. This device preferably also contains a valve that only allows the cyclodextrin to go out of the container and doesn't allow external elements to enter the container.

In a preferred embodiment, this device is a bag on valve aerosol system, consisting of an aerosol valve with a welded bag, the (2,6-di-O-methyl)-beta-cyclodextrin being placed inside the bag and the propellant is filled in the space between bag and can. The bag may be made in any suitable material. In particular, it can be made in laminated aluminum (3 or 4 layers depending on product requirements). The aerosol can is a standard one, and can be made in aluminum or tin. The shape of the can may be of any sort, although it is preferred when it has the shape of a cylinder. The propellant may be any of type of the art, and is preferably nitrogen or compressed air.

The device of the invention thus consists in a device that contains:
- A container filled with a liquid composition containing (2,6-di-O-methyl)-beta-cyclodextrin
- A nasal tip to be placed in the nasal cavity
- A valve or a pump to dispense a predetermined amount of (2,6-di-O-methyl)-beta-cyclodextrin upon action of the patient, wherein the liquid composition is dispensed as a spray at the exit of the nasal tip.

As indicated above, in a preferred embodiment, the container is a bag that is itself contained in a can.

In a specific embodiment, the liquid composition (which is an integral part of the device herein disclosed) is composed of (2,6-di-O-methyl)-beta-cyclodextrin and water only. In another embodiment, the liquid composition is composed of (2,6-di-O-methyl)-beta-cyclodextrin and an isotonic solution as disclosed above. In another embodiment, the liquid composition is composed of (2,6-di-O-methyl)-beta-cyclodextrin and an hypotonic saline solution. In another embodiment, the liquid composition is composed of (2,6-di-O-methyl)-beta-cyclodextrin and an hypertonic saline solution.

In a preferred embodiment, the concentration of (2,6-di-O-methyl)-beta-cyclodextrin in the composition is such as to deliver unit dose (for each take) compatible with the therapeutic regimen determined by the physician.

The valve and pump are designed so as to deliver a volume of about between 50 and 200 µl (preferably around 100 µl) for each squeeze on the pump.

The invention also relates to a composition containing (2,6-di-O-methyl)-beta-cyclodextrin solubilized in water as a medicament.

In another embodiment, mention is made of a composition containing (2,6-di-O-methyl)-beta-cyclodextrin solubilized in an isotonic saline solution as a medicament.

In another embodiment, mention is made of a composition containing (2,6-di-O-methyl)-beta-cyclodextrin solubilized in a hypotonic saline solution as a medicament.

In another embodiment, mention is made of a composition containing (2,6-di-O-methyl)-beta-cyclodextrin solubilized in a hypertonic saline solution as a medicament.

In one embodiment, the compositions do not contain preservative and thus only contain the cyclodextrin and the buffer (water or saline solution). In this embodiment, sterilization may be performed by gamma rays. Alternatively, one can use materials and vials that are sterile and are filled in a sterile environment. Such sterile filling may be performed by passing the solution through a filter with a cut-off retaining any bacteria or fungus that could be present in the cyclodextrin composition. It is to be noted that heat-sterilization of the product is generally not used as this may degrade the cyclodextrin.

In another embodiment, the compositions contain preservatives. Such preservatives are known and common in the art, are nontoxic, stable, compatible, inexpensive. A preservative may be an antimicrobial compound (such as sorbic acid and salts (sorbates), benzoic acid and salts (benzoates), hydroxybenzoate and derivatives, or other agent known in the art), an antioxidant (such as ascorbic acid, sodium ascorbate, butylated hydroxytoluene, butylated hydroxyanisole, gallic acid and gallate (sodium or propyl), tocopherols or others). It may also be a sequestering or chelating agent (such as disodium EDTA, citric acid (and citrates), tartaric acid, or lecithin).

The invention also relates to a method for treating a prion disease, comprising the administration to a patient in need thereof of a therapeutic amount of (2,6-di-O-methyl)-beta-cyclodextrin preferably by nasal administration. The invention also relates to a method for treating a patient with a prion disease comprising the administration to said patient in need thereof of a therapeutic amount of (2,6-di-O-methyl)-beta-cyclodextrin preferably by nasal administration. Such administration is preferably performed in such a way that at least 30%, more preferably at least 40% of the dose nasally administered reaches the olfactory nerves. The compositions are as disclosed above. A therapeutic amount (or effective amount) is an amount sufficient to effect beneficial or desired results, such as clinical results, and an "effective amount" depends upon the context in which it is being applied. In the context of administering DIMEB to a patient with a disease herein disclosed, an effective amount of an agent is, for example, an amount sufficient to achieve an improvement of the cognitive abilities of the patients as compared to the response obtained without administration of the agent. It can also be an amount that stops or slows the cognitive deterioration of the patients. It can be an amount that allows regression of biological markers associated with the specific disease.

The invention also relates to a method for manufacturing a device that can be used for a therapeutic purpose, comprising the steps of filling the container of a nasal delivery device with a sterile isotonic composition containing (2,6-di-O-methyl)-beta-cyclodextrin, and optionally (but preferably) sterilizing the device with the filled container. In the preferred embodiment, said container is a bag-in-can device (bag-on-valve technology). In this embodiment, the container is a flexible bag within a rigid can.

It is to be noted that other modes of administration (oral, injection, transdermal, sub-lingual...) are contemplated, in addition of the more described nasal mode of administration.

### Description of the Figures

Figure 1: A. Side view of the head of a patient, showing the angle of introduction of the device in the nostril with regards to the vertical line. B. Top view of the head of a patient, showing the angle of introduction of the device in the nostril with regards to the midsagittal plane.
Figure 2: Mean (±SEM) relative lag phase of RT-QuIC reactions illustrating the delay of conversion of hamster recombinant PrP by Prions from sCJD-MM1 patients (N=4-5 in quadruplicates), for two different numbers of seeded cells: A.: 10⁻⁶ seeded cells; B. 10⁻⁷ seeded cells.
Figure 3: Mean (±SEM) cell viability normalized to control in primary cultures of murine cerebellar granule neurons treated with DIMEB at concentrations from 10 to 1500 µM (N=2 in sixplicate; **** p<0.0001).
Figure 4: Mean (±SEM) PrP^{res} accumulation in murine primary cultures of cerebellar granule neurons treated with DIMEB at 250 µM (A.) or 500 µM (B.) (At least n=3 in duplicate; * p<0.05).
Figure 5: Mean (±SEM) touch response at the tail in young and old C. *elegans* animals, expressing GFP and human PrP as controls or E200K mutated PrP treated with DIMEB at 250 or 500 µM *versus* vehicle (n=3; ** p<0.01).
Figure 6: Mean (±SEM) number of mechano-sensitive neurons of C. *elegans* animals, expressing human PrP as control or E200K mutated human PrP treated with DIMEB at concentrations from 1 to 500 µM *versus* vehicle (NS: not significant; ** p<0.01; **** p<0.0001).
Figure 7: Kaplan-Meier survival analysis of 22L mouse-adapted scrapie strain mice treated intranasally with DIMEB at 0.032 mg/day *versus* vehicle from 120 dpi to the terminal stage of the disease (Left line: vehicle; Right line: DIMEB; Gehan-Breslow-Wilcoxon test: *p* = 0.0137). The dotted lines represent the 50% lethality for control mice (left dotted line) ot DMIEB-treated mice (right dotted line).

### Examples

### Example 1 - Use of DIMEB in an acellular model of prion diseases studying the conversion and aggregation of recombinant PrP^{C} by human PrP^{Sc} isolates

### Model:

In Prion diseases, the infectious agent is mainly, if not solely, composed of abnormal PrP^{Sc} and is capable of converting cellular PrP^{c} into PrP^{Sc} in an autocatalytical manner.

Among recent methods developed to amplify prions *in vitro,* the real-time quaking-induced conversion (RT-QuIC) assay allows the sensitive detection of prion seeding activity in numerous tissues of animal and human origins. The RT-QuIC assay relies upon the conversion of recombinant prion protein (recPrP) by prion-associated seeds into amyloid fibrils in presence of an amyloid-sensitive dye, thioflavine T (ThT). The incorporation of ThT within elongating fibrils is monitored in a multiwell plate in real time.

### Material and method:

- Hamster recombinant full length PrP are incubated with diluted brain homogenates (=seeds) from patients with sporadic Creutzfeldt-Jakob disease characterized by MM1-type PrP (sCJD-MM1) in the presence of DIMEB or vehicle. Conversion of PrP^{c} to PrP^{Sc} is measured by thioflavin T fluorescence every 15 min (N=4-5 quadruplicates).
- DIMEB concentrations tested: 10, 25, 50, 75, 100, 250 and 500 µM.
- The statistical significance of the difference between lag phases of analyzed groups was assessed using the non-parametric, unpaired t-test with unequal variance (Welch correction).

### Results:

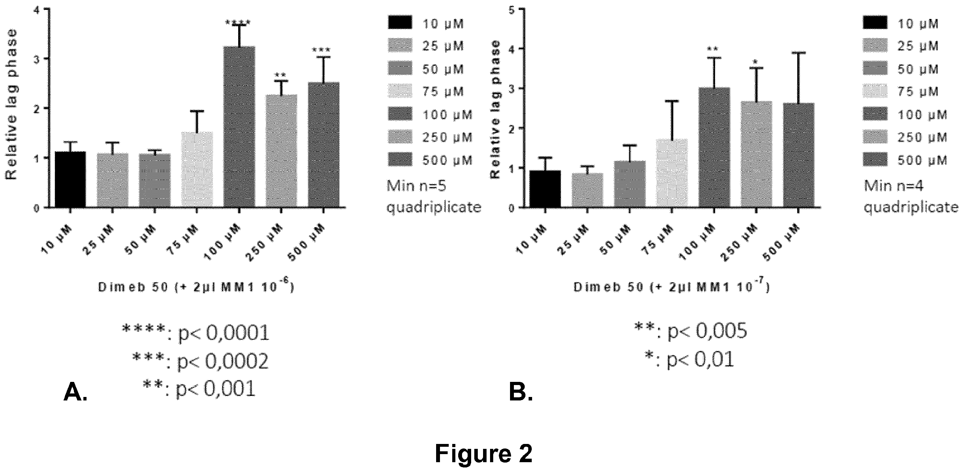

DIMEB significantly increased the lag phase (time for the reaction to start), with a maximum effect at 100µM (bell effect observed). The effect is more marked with the seeding concentration at 10⁻⁶ (Figure 2).
Thus, DIMEB has an inhibitory effect on the conversion and aggregation of recombinant PrP^{c}.

### Example 2 - Use of DIMEB in a cellular model of prion diseases

### Model:

Primary cultures of cerebellar granule neurons (CGN) derived from Tg650 transgenic mice overexpressing human PrP^{C}, with a methionine at codon 129, infected with human PrP^{Sc} isolates from patients with sCJD-MM1. The sCJD-MM1 isolate corresponds to the most common form of human prion diseases.

These CGN cultures shows a progressive accumulation of protease resistant fragment (PrP^{res}) after exposition to human prion isolates. Molecular features of PrP^{res} are evidenced by Western Blot after proteinase K digestion. PrP^{res} accumulation was associated with the detection of PrP aggregates in infected cells.

### Material and method:

- Primary Neuronal Cells Cultures: CGNs were dissociated from the cerebellum of 6- to 7-day-old tg650 mice and cultured on plastic culture wells precoated with poly-d-lysine in Dulbecco's Modified Eagle's Medium-Glutamax I high glucose supplemented with penicillin and streptomycin, fetal calf serum, potassium chloride, and N2 and B27 supplements. Every week, the medium was supplemented with glucose and the antimitotics uridine and fluorodeoxyuridine were added to reduce astrocyte proliferation.
- Prion Infection: Primary neuronal cultures were exposed to sonicated brain homogenates from patients with sCJD-MM1 48 hours after plating and for 4 days. Neurons were then maintained for up to 5 weeks.
- Cell viability assay: at 17 days post-exposure (dpe), a tetrazolium (MTT) assay to allow quantification of cell survival and/or proliferation.
- PrP^{res} Detection: at 28 dpe, PrP^{res} levels were detected by Western blot using the anti-PrP monoclonal antibody 3F4 (IgG2a, epitope 109-112), which detect the 3 PrP^{Sc} glycoforms, and electrochemiluminescence was measured using a semi-quantitative method.
- Treatment:
   ∘ In toxicity experiments, cultures were treated with various concentrations of DIMEB (from 0 to 1500 µM) or with vehicle only from 14 to 17 dpe. A MTT assay was performed at 17 dpe (N=2 in sixplicate).
   ∘ Efficacy experiments: cultures were treated with various concentrations of DIMEB (from 0 to 500 µM) or with vehicle only from 14 to 28 dpe. A PrP^{res} detection was performed at 28 dpe (N=3 in duplicate).
- A Mann-Whitney test was used to compare 2 groups (efficacy experiments) and a 1-way analysis of variance (Dunnett's test) to compare DIMEB concentrations (toxicity experiments) to controls.

### Results:

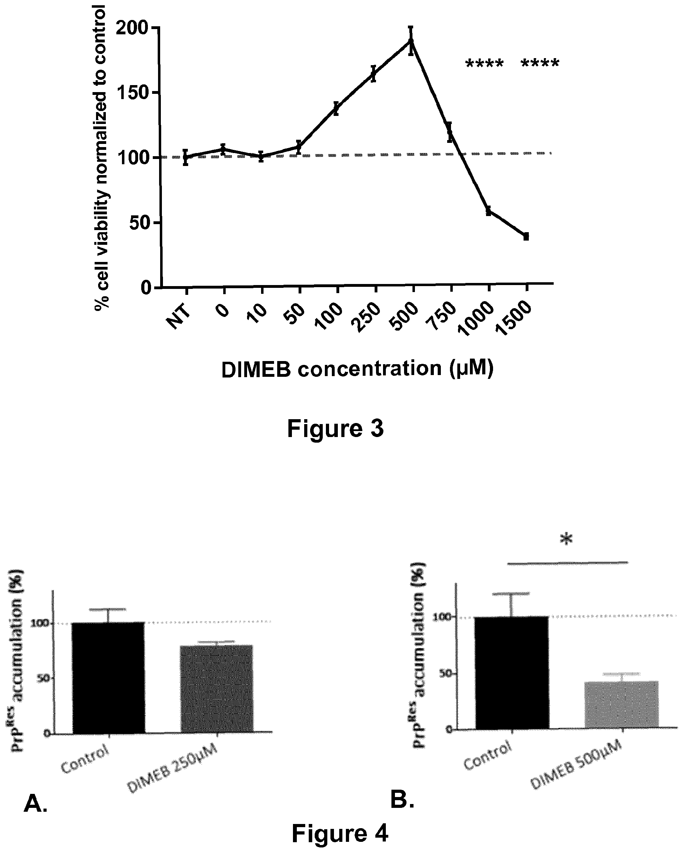

Toxicity was observed (MTT test) for DIMEB doses higher than 500 µM (p<0.0001). An increased signal was observed for doses between 50 and 500 µM suggesting astroglial proliferation (Figure 3).
DIMEB at 500 µM significantly decreased PrP^{Sc} accumulation (p<0.05), revealed by proteinase K digestion and WB (Figure 4).

### Example 3 - Use of DIMEB in an in vivo model of Prion diseases - C. elegans

### Model:

*C. elegans* is a nematode with a well-defined neuronal system (neurons lineage, neuronal connections, neuro-transmitters...) showing a strong gene homology with mammals including humans, devoid of human PrP homolog, and has proven to be a powerful genetical model to study the molecular mechanisms of cell toxicity notably due to a gain of function mechanism.

Transgenic worm lines express human PrP with an insertional mutation that produced an abnormal PrP sharing cellular and biochemical properties with that observed in patients with the same pathogenic mutation.

The line used in the experiments expressed human PrP with the most frequent pathogenic PrP mutation worldwide (E200K) (responsible for inherited Creutzfeldt-Jakob disease) in the mechanosentive neuronal system.

The expression of E200K mutated PrP in the mechanosensory neurons led to a progressive disorder associating a loss of mechano-sensory neurons, a loss of touch response with a global motility deficiency and a decrease of viability. Neurons expressing E200K PrP showed PrP clustering and axonal blebbing.

### Material and method:

- Transgenic lines were generated by microinjection in the gonads of the animals of various *PRNP* genotype plasmids (*Pmec-7::PRNPs*) in association with a GFP fluorescent marker plasmid (*Pmec-7::gfp*). Animals were isolated to select the new transgenics transformants with the presence of the GFP marker signal in the mechanosensitive system.
- Drug assay: Synchronized L1 larvae were incubated with DIMEB (0 to 500 µM) until they reached young adulthood (3 days) or old adults (6 days).
- Mechanosensory assays: Touch tests involved scoring for the response to light touch at the tail by using a fine hair.
- Neuronal loss: Number of GFP positive PLM neurons.
- An analysis variance with a one-way ANOVA followed with Tukey multiple comparison test.

### Results:

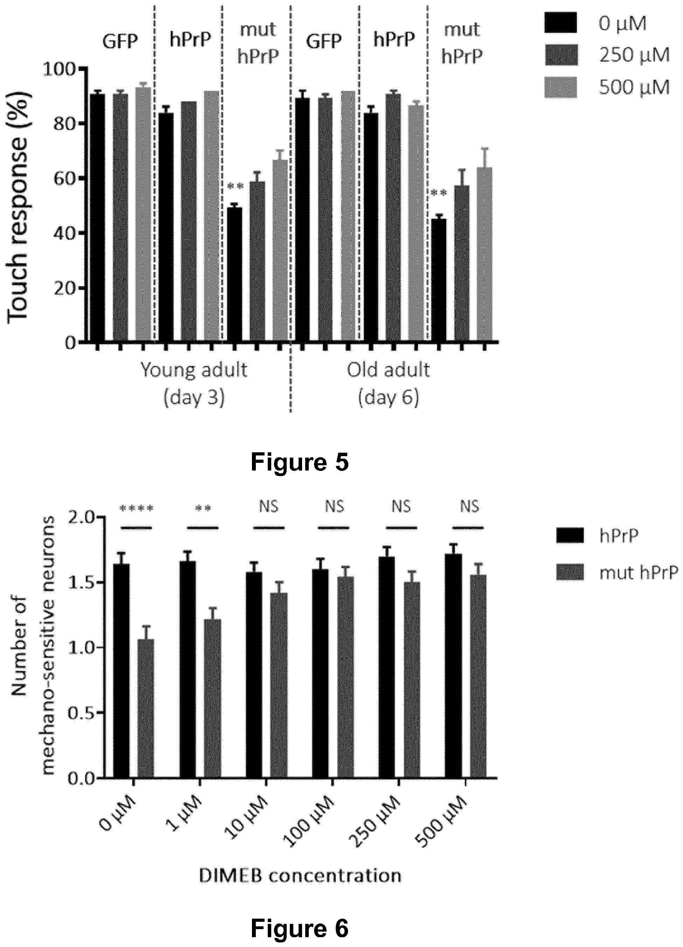

DIMEB at 500 µM significantly inhibited the loss of function of the mechano-sensory neurons in the touch response test (Figure 5).
DIMEB, at concentrations from 10 µM, significantly restored the number of mechano-sensory neurons (Figure 6).

### Example 4 - Use of DIMEB in an in vivo model of prion diseases

Model: C57BL/6 mice intracerebrally inoculated with the mouse-adapted scrapie strain 22L is a well-known scrapie model and is widely-used for testing anti-prion compounds. Following a 4-5-month incubation period, inoculated mice begin to develop progressive clinical signs of neurological illness including ataxia (uncoordinated movement), tail rigidity, and kyphosis (hunched back). After this around 7-day symptomatic phase, mice will succumb to prion disease. This assay is remarkably robust. The brains of prion-infected mice recapitulate all of the neuropathological hallmarks of prion diseases in humans and animals, including spongiform degeneration (vacuolation of the brain), deposition of misfolded PrP, and prominent astrocytic gliosis. Furthermore, PK-resistant PrP^{Sc} can readily be detected in brain homogenates prepared from clinically ill prion-inoculated mice.

### Material and method:

- Mice
   ∘ Inocula with correct PrP^{res} level were prepared extemporaneously from whole brains of C57BL/6 mice infected with experimental 22L scrapie strain.
   ∘ 7- to 8-week-old C57BL/6 mice were intracerebrally inoculated with the 22L mouse-adapted scrapie strain.
      - Mice were daily monitored and the evolution of the disease was determined using an evaluation scale (0: none; 1: discreet; 2: weak; 3: medium; 4: strong), based on the following parameters: general state, cerebellar ataxia, paresis, kyphosis and prostration state. Sacrifice was realized at stage 4.
- Treatment
   ∘ Bi-daily intranasal administration.
   ∘ 3 groups (N = 12/group): non-treated, vehicle and DIMEB (0.032 mg/day).
   ∘ Duration: from 120 days post-infection to the terminal stage of the disease. This therapeutic window is considered as almost curative (close to the symptomatic phase: 135-140 dpi). In most other studies, investigational treatments are preventive.
- Western blotting: Brain digested samples were loaded onto gels for migration and then proteins were transferred to nitrocellulose membranes. PrP detection was performed using anti-PrP monoclonal antibody sha31 (lgG1, epitope 148-159) at a dilution of 1/5000. Horseradish peroxidase-conjugated goat antimouse secondary antibody was used at a dilution of 1/10000.

### Results:

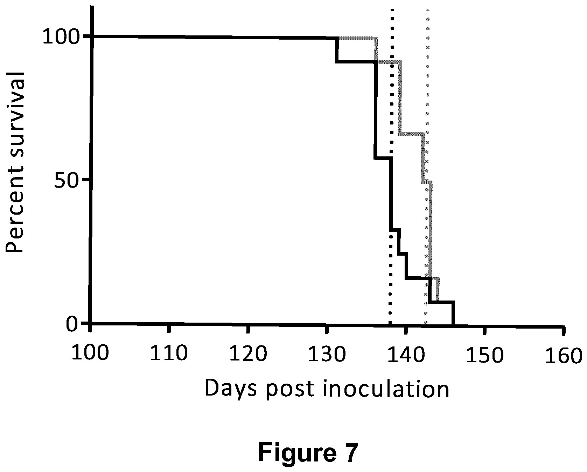

DIMEB (blue) increased significantly the survival of the 22L mouse-adapted scrapie strain mice in comparison to vehicle (black) (142.5 dpi vs 138 dpi; Gehan-Breslow-Wilcoxon test: *p* = 0.0137) (Figure 7).
When DIMEB was administered from 120 dpi and mice euthanized at the terminal stage of the disease, brain analysis did not show any difference in PrP^{res} accumulation between the groups (data not shown).

### Example 5 - use in supine position

Experiments were performed using an artificial nose to determine the amount of a dose that can reach the back structure of the nose, and in particular the neurosensorius olfactorius.

Different positions were tested with a patient standing (nose at 0° of the vertical axis), lying down with the head in the extension of the body (nose at 90° of the vertical axis) or lying down with the chin raised (the head is at 45° of the lying body axis, the position of the nose being at 135° of the vertical axis).

The best position (highest percentage of therapeutic delivers in the neurosensorius olfactorius) was observed when the patient is lying on his back, when the angle of the dispensing device is about 60° relative to line perpendicular to the horizontal plane determined by the lying body (coronal plane). It is further preferred when the dispensing device is introduced in the nostril with an angle of about 5° relative to the vertical plane (median or midsagittal plane).

Figure 1 shows the optimal delivery mode of DIMEB in the patient's nostrils.

Table 1 shows the percentage of deposition of DIMEB in various regions of the nose, in an artificial nose model, according to the angle of application of the spray. The dispensing device was bag-on-valve spray (CPS pump, Aptar), and the dispensed product was DIMEB + fluorescein, making it possible to quantify the amount reaching the different regions of the nose. The device was manually actuated and each pump was actuated only once in each nostril.

**Table 1. Percentage of deposition of product**

| | Nose+ Nasal valve | Floor | Turbinates | Olfactory zone (Ethmoids) | Rhino-pharynx | Filtre |
|---|---|---|---|---|---|---|
| 30° | 6.2% | 0.0% | 73.5% | 19.9% | 0.3% | 0.0% |
| 60° | 23.1% | 0.0% | 33.6% | 43.4% | 0.3% | 0.0% |

It was thus possible to deliver 40 % of the dose on the olfactory mucosa, thereby guaranteeing that the dose will reach the brain. This is particularly efficient when a bag-in-can device is used for delivery.

Using other types of device, it was shown that they were all able to deliver at least 18% of the product on ethmoids, with low deposition in the nose and the nasal valve, thus ensuring a good coverage of the entire internal nasal cavity. This is obtained whatever the formulation concentration of DIMEB (tested with DIMEB 0.4% and 6%).

## Claims

1. (2,6-di-O-methyl)-beta-cyclodextrin for its use for the treatment of a prion disease.

2. (2,6-di-O-methyl)-beta-cyclodextrin for its use according to claim 1, in a nasal composition, for the treatment of a prion disease by nasal administration.

3. (2,6-di-O-methyl)-beta-cyclodextrin for its use according to claim 1 or 2, wherein the prion disease is selected from the group consisting of Creutzfeldt-Jakob disease (CJD), Iatrogenic Creutzfeldt-Jakob disease (iCJD), Variant Creutzfeldt-Jakob disease (vCJD), Familial Creutzfeldt-Jakob disease (fCJD), Sporadic Creutzfeldt-Jakob disease (sCJD), Gerstmann-Straussler-Scheinker syndrome (GSS), Fatal familial insomnia (FFI), Kuru, Familial spongiform encephalopathy.

4. (2,6-di-O-methyl)-beta-cyclodextrin for its use according to any one of claims 2 to 3, wherein the nasal composition contains only (2,6-di-O-methyl)-beta-cyclodextrin in an isotonic composition.

5. (2,6-di-O-methyl)-beta-cyclodextrin for its use according to claim any one of claims 2 to 4, wherein the patient is lying on its back (i.e. is in supine position) during nasal administration.

6. (2,6-di-O-methyl)-beta-cyclodextrin for its use according to any one of claims 2 to 5, wherein the (2,6-di-O-methyl)-beta-cyclodextrin is delivered though spraying.

7. (2,6-di-O-methyl)-beta-cyclodextrin for its use according to any one of claims 1 to 5, wherein the daily amount of cyclodextrin delivered to the patient is comprised between 5 and 50 mg.

8. A device for nasally administering (2,6-di-O-methyl)-beta-cyclodextrin, comprising a container containing a liquid composition containing (2,6-di-O-methyl)-beta-cyclodextrin, and a nasal tip allowing spray delivery of the composition.

9. The device of claim 8, wherein the liquid composition is composed of (2,6-di-O-methyl)-beta-cyclodextrin and water only.

10. The device of any one of claims 8 to 9, wherein the dose delivered by the device upon action is comprised between 50 and 200 µl.

11. The device of any one of claims 8 to 10, which is a bag-in-can device.

12. Composition containing (2,6-di-O-methyl)-beta-cyclodextrin solubilized in an isotonic solution as a medicament.

13. A method for manufacturing a device for the treatment of a prion disease by nasal administration, comprising the step of filling the container of a nasal delivery device with a sterile isotonic composition containing (2,6-di-O-methyl)-beta-cyclodextrin and optionally preservatives.

14. The method of claim 13, wherein the sterile isotonic composition is a potassium sodium phosphate buffer 10 mM.

15. The method of claim 13 or 14, wherein the composition contains EDTA as a preservative.

16. The method of any one of claims 13 to 15, wherein the device is a bag-in-can device and the container is a flexible bag within a rigid can.

17. The method of any one of claims 13 to 16, wherein the step of filling is sterilely performed.
